# EUROPEAN PATENT APPLICATION

(11) **EP 1 738 758 A1**
(43) Date of publication of application: **03.01.2007**
(21) Application number: 06112020.0
(22) Date of filing: 30.03.2006
(51) Int. Cl.: A61K 31/327, A61K 31/13

(54) **Antimicrobial combination of amine and oxygen compounds**

(30) Priority: 13.05.2005 US 129431
(71) Applicant: ECOLAB, INC., St. Paul, MN 55102-1390 (US)
(72) Inventor: Stingl, Carola, 40597, Düsseldorf (DE); Meyer, Bernhard, 40822, Mettmann (DE)
(74) Representative: Sternagel, Fleischer, Godemeyer & Partner

(57) **Abstract**

The present invention relates to a two-part antimicrobial system having an amine part and an oxygen part, where the two parts are mixed prior to use to form an improved antimicrobial composition. Also disclosed are antimicrobial compositions and methods of making, and using the antimicrobial compositions.

## Description

### FIELD OF THE INVENTION

The present invention relates to a two-part antimicrobial system having an amine part and an oxygen part, where the two parts are mixed prior to use to form an improved antimicrobial composition. Also disclosed are antimicrobial compositions and methods of making, and using the antimicrobial compositions.

### BACKGROUND

Many diseases arise from the growth and spread of microorganisms that can affect all aspects of life, from human health, to animal health, to food and water safety, to the safety to the environments we live in. Antimicrobial agents have found widespread application in all these areas. Hospitals perform rigorous programs to disinfect and sterilize their environments. Consumer homes are replete with disinfectant hand cleaners, sprays, hard surface cleaners, disinfectant wipes, and fruit and vegetable washes. Antimicrobial agents are widely used on farms where the difference between healthy and sick animals can mean the difference between profitability and loss. Many antimicrobial compositions are known and used in these and other applications. Two separate examples of antimicrobial compositions include amine compositions and oxygen compositions.

There are many varieties of microorganisms having various levels of resistance to disinfectants or antimicrobial agents. Examples of microorganisms, in descending order of resistance, include prion, bacterial spores, mycobacterium, nonlipid small viruses, fungi, vegetative bacteria, and lipid or medium sized viruses. Known antimicrobial agents may be effective against some of these microorganisms, but not all microorganisms, with viruses and spores being especially difficult to kill.

That is against this background that the present invention has been made.

### SUMMARY

It has been discovered that a two part antimicrobial system having an amine part and an oxygen part provides an improved antimicrobial composition where the two parts are mixed prior to use. Specifically, the resulting antimicrobial composition has increased efficacy over a wide variety of microorganisms, including spores and viruses. The efficacy of the amine part and the oxygen part together is greater than the efficacy of either the amine part or the oxygen part by itself.

Accordingly, in some embodiments, the present invention relates to a two part antimicrobial system having an amine part and an oxygen part. In some embodiments, the present invention relates to an improved antimicrobial composition having both an amine component and an oxygen component that provide a synergistic effect. In some embodiments, the present invention relates to methods of making and methods of mixing a two part antimicrobial system to form an improved antimicrobial composition having an amine component and an oxygen component that provide a synergistic effect. Finally, in some embodiments, the present invention relates to methods of using an antimicrobial composition having an amine component and an oxygen component that provide a synergistic effect.

These and other embodiments will be apparent to those of skill in the art and others in view of the following detailed description of some embodiments. It should be understood, however, that this summary, and the detailed description illustrate only some examples of various embodiments, and are not intended to be limiting to the invention as claimed.

### DETAILED DESCRIPTION OF SOME EMBODIMENTS

### Definitions

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the term "about" may include numbers that are rounded to the nearest significant figure.

Weight percent, percent by weight, % by weight, wt %, and the like are synonyms that refer to the concentration of a substance as the weight of that substance divided by the weight of the composition and multiplied by 100.

The recitation of numerical ranges by endpoints includes all numbers subsumed within that range (e.g. 1 to 5 includes 1, 1.5, 2, 2.75, 3, 3.80, 4 and 5).

As used in this specification and the appended claims, the singular forms "a," "an," and "the" include plural referents unless the content clearly dictates otherwise. Thus, for example, reference to a composition containing "a compound" includes a mixture of two or more compounds. As used in this specification and the appended claims, the term "or" is generally employed in its sense including "and/or" unless the content clearly dictates otherwise.

The use of the terms "antimicrobial" in this application does not mean that any resulting products are approved for use as an antimicrobial agent.

### Compositions

As previously discussed, it has been discovered that a two part antimicrobial system having an amine part and an oxygen part provides an improved antimicrobial composition where the two parts are mixed prior to use. Specifically, the present invention has the advantage of having increased efficacy over a wide variety of microorganisms, including spores and viruses. The efficacy of the amine part and the oxygen part together is greater than the efficacy of either the amine part or the oxygen part by itself. Further, the present invention allows for increased levels of the oxygen compound, and in particular peracids, without the strong odors typically associated with peracids, by controlling the pH of the composition.

Amine compositions and oxygen compositions are generally not considered compatible together because the oxygen composition will typically oxidize the amine composition over time, rendering both the amine composition and the oxygen composition ineffective as antimicrobial agents. However, it has been discovered that a combination of specific amine compositions and an oxygen composition are compatible for a limited period of time and have a synergistic effect on efficacy against microorganisms, without rendering both components ineffective. Specifically, the combination has been found to be effective for a time period of at least 15 minutes, but up to 4 hours.

Accordingly, in some embodiments, the present invention relates to a two part antimicrobial system having an amine part and an oxygen part. In some embodiments, the present invention relates to an improved antimicrobial composition having both an amine component and an oxygen component that provide a synergistic effect. In some embodiments, the present invention relates to methods of making and methods of mixing a two part antimicrobial system to form an improved antimicrobial composition having an amine component and an oxygen component that provide a synergistic effect. Finally, in some embodiments, the present invention relates to methods of using an antimicrobial composition having an amine component and an oxygen component that provide a synergistic effect.

The two part antimicrobial system includes both an amine part, or an amine composition, and an oxygen part or an oxygen composition. The amine part includes an amine having antimicrobial activity. The oxygen part includes an oxygen having antimicrobial activity. Both the amine part and the oxygen part may include additional functional ingredients that increase the effectiveness of the composition or provide an additional functional benefit.

The individual compositions may be a concentrate or a use composition, and when combined may form a concentrate or a use composition. The concentrate refers to the composition that is diluted to form the use composition. The concentrate may be a solid, liquid, emulsion, cream, paste, gel, powder, tablet, or the like. The concentrate is preferably a liquid. The use composition refers to the composition that is applied to a surface to kill microorganisms. For example, the concentrate composition may be diluted with water to a 1-4% use composition (0.005 wt.% to 2 wt.% active) and then applied to a surface. It may be beneficial to form the composition as a concentrate and then dilute it to a use composition upon mixing the two parts at the point of use. The concentrate is often easier and less expensive to ship than the use composition.

The present use compositions preferably have a pH from about 2 to about 10, from about 3 to about 9, and from about 3 to about 6.

### Amine Compositions

The composition includes an amine or amine derivative having antimicrobial activity in an amine part. Examples of amines or amine derivatives having antimicrobial activity include the reaction product of alkylpropylene diamine with glutamic acid called GLUCOPROTAMIN, N-cocotrimethylenediamine, dodecylmorpholine-N-oxide, alkylpropylenediamine, and N,N-Bis(3-aminopropyl)dodecylamine, commercially available as Lonzabac 12 (Lonza). The amine is preferably GLUCOPROTAMIN. An example of a suitable formulation with an amine includes that sold under the tradename INCIDIN PLUS, commercially available from Ecolab Inc.

The amine is preferably present in the amine part so that when combined with the oxygen part, the active is present in the resulting use composition from about 0.01 to about 5 wt.%, from about 0.1 to about 2 wt.%, and from about 0.25 to about 2 wt.%.

### Oxygen Compositions

The composition includes an active oxygen species in an oxygen part. Suitable active oxygen species are those oxygen based compounds and their derivatives that have antimicrobial activity. Examples of suitable oxygen compounds include peroxygen compounds such as organic and inorganic peroxides, peracids, peresters, and mixtures thereof. Non-limiting examples of inorganic peroxides include: hydrogen peroxide and its salts, and other inorganic acids or salts of percarbonates, persulfates, and perborates. Non-limiting examples of organic peroxides include: benzoyl peroxide, tert-butyl benzoyl peroxide, and other alkyl and/or aryl peroxides. Non-limiting examples of peracids include: performic acid, peracetic acid, perlactic acid, perglycolic acid, chloroperbenzoic acid, perheptanoic acid, peroctanoic acid, perdecanoic acid, percitric acid, and perbenzoic acid. Non-limiting examples of perester peracids include: monoester peracids derived from diacids or mono-ester diacids or diesters (e.g. such as adipic, succinic, glutaric, sebacic, or malonic acids/esters and mixtures thereof). It is also possible to utilize oxidants capable of generating active oxidizing or oxygen species including oxygen, ozone, chlorine dioxide, chlorine, sulfur dioxide, sulfur trioxide, and singlet oxygen generators (e.g. paratoluidine blue, methylene blue), and mixtures thereof. The preferred oxygen compounds are peroxygen compounds including hydrogen peroxide and peracids, and specifically peracetic acid. An example of a suitable oxygen composition includes that sold under the tradename PERESAL, commercially available from Ecolab Inc.

The oxygen compound is preferably present in the oxygen part so that when combined with the amine part, the active is present is the resulting use composition from about 0.005 to about 0.5 wt.%, from about 0.01 to about 0.1 wt.%, and from about 0.02 to about 0.1 wt.%.

Various optional ingredients may also be present in the amine part, the oxygen part, or both the amine part and the oxygen part. Such ingredients include (but are not limited to) wetting agents, builders, complexing agents, textual modifiers, film-forming polymers, surfactants, colorants, fragrances, and mixtures thereof. The wetting agents facilitate contact of the disinfecting composition with the skin or surface, and can be selected from those materials recognized to provide this effect, in both identity and amount. Builders and complexing agents help boost the cleaning performance of surfactant systems and are typically associated with materials that are capable of complexing with polyvalent cations, such as calcium and magnesium. Textural modifiers are those materials which primarily affect the body of the mixed disinfecting composition in terms of retention, flow and lubricity. These include thickening agents such as alkyl celluloses, alkoxy celluloses, xanthan gum, guar gum, and polyacrylamide derivatives, of which the polymer of 2-acrylamido-2-methylpropane sulfonic acid is a preferred example. Inorganic thickening agents include hectorite, synthetic hectorite, magnesium aluminum silicate, bentonite, montmorillonite, and amorphous silicon dioxide. Thickening can also be achieved by the combination of selective surfactant classes. Other textural modifiers include lanolin derivatives, acyl lactylates, polyethylene glycol, glyceryl esters, and mixtures thereof. Skin conditioning and skin healing agents include glycerin, sorbitol, pyrrolidone carboxylic acid, mineral oils, silicone oils, protein hydrolysates, petrolatum, hydrocarbon emollient alcohols and esters, allantoin, aloe vera extracts, and urea. Film-forming polymers include the above-referenced polyacrylamides, as well as the class of poly(vinyl alcohols/vinyl acetates), polyurethanes, chitosan, polyvinyl pyrrolidone, and polyvinyl pyrrolidone copolymers.

### Methods of Mixing and Application

The present invention may be combined in several ways. For example, the amine part and the oxygen part may be combined manually, by dosing equipment, and by a two-part spraying system. The amine part and the oxygen part may be combined to create a desired ratio of active amine species to active oxygen species. Examples of suitable amine to oxygen ratios include 1:100 to 1:1 to 100:1.

The use composition may be applied to a surface in a variety of ways including wiping (for example using a non-woven wipe embedded with a use composition), spraying, and foaming.

The use composition may be applied to a variety of substrates. For example, the substrate may be any surface or material in need of, or that would benefit from, such disinfection, including (but not limited to) skin or tissue, as well as body fluids and mucosal membranes. In addition, the substrate may be any surface of a food product, such as meat, fish, fruits and vegetables. The substrate may also include food contact surfaces, and nonfood contact surfaces in food processing plants. The substrate may include any hard surface, such as (but not limited to) floors, walls, countertops, containers, instruments and/or equipment found in homes, hospitals, and manufacturing facilities. In a specific application, the hard surfaces may include housing and equipment surfaces in animal rearing and production environments. Materials that may benefit from disinfection include, for example, process waters, such as flume waters, cooling tower waters, livestock drinking waters, equipment, and facility cleaning solutions.

For a more complete understanding of the invention, the following examples are given to illustrate some embodiment. These examples and experiments are to be understood as illustrative and not limiting. All parts are by weight, except where it is contrarily indicated.

### EXAMPLES

### Example 1

Example 1 tested the ability of the combination of the amine part plus the oxygen part to kill spores. For this example, three combinations of INCIDIN PLUS (25% Glucoprotamine) commercially available from Ecolab Inc., and PERESAL (4.5% peracetic acid) commercially available from Ecolab Inc. were tested against a *Bacillus subtilis* (DSM 347) spore suspension at 20 °C in a suspension test according to the European draft standard document CEN TC 216 WG1 N 176.

**Table 1 Reductions Against Bacillus subtilis**

| **Product** | **Volume Percent** | **30 Minutes** | **60 Minutes** |
|---|---|---|---|
| Incidin Plus | 1.0* | 2.07 x 10³ | 4.3 x 10³ |
| Peresal | 0.5 | | |
| Incidin Plus | 1.0 | >3.2 x 10⁵ | >3.2 x 10⁵ |
| Peresal | 1.0 | | |
| Incidin Plus | 1.0 | >3.2 x 10⁵ | >3.2 x 10⁵ |
| Peresal | 2.0 | | |

| | | | |
|---|---|---|---|
| *1% of INCIDIN PLUS includes 2600 ppm of GLUCOPROTAMIN. 1% of PERESAL includes 450 ppm of peracetic acid. | | | |

The lower concentration of the Peresal (0.5%) with the Incidin Plus was less effective at killing all of the spores. A 1% solution of the Peresal with a 1% solution of the Incidin Plus was effective of killing substantially all of the spores as was a 2% solution of the Peresal and a 1% solution of Incidin Plus.

### Example 2

Example 2 tested the ability of the combination of the amine part and the oxygen part to kill viruses. For this example, various combinations of INCIDIN PLUS and PERESAL were tested against the Polio virus (Mahoney Strain) in a suspension test according to the guidelines of the German Society for the Prevention of Viral Disease (DVV).

**Table 2 Log Reductions Against the Polio Virus (Mahoney Strain)**

| **Product** | **Volume Percent** | **pH** | **Soiling** | **Virus** | **Log Reduction Factors** | |
|---|---|---|---|---|---|---|
| | | | | | **30 Minutes** | **60 Minutes** |
| Incidin Plus | 1* | 5.27 | No soil | Polio | 0.4 | 2.0 |
| | | | Fetal cow serum | | 0.9 | 1.5 |
| Peresal | 0.5 | | Bovine serum albumin | | 1.0 | 1.5 |
| Incidin Plus | 1 | 4.64 | No soil | | ≥4.5 | ≥4.5 |
| | | | Fetal cow serum | | ≥4.5 | ≥4.5 |
| Peresal | 1 | | Bovine serum albumin | | ≥4.5 | ≥4.5 |
| Incidin Plus | 1 | 4.07 | No soil | | ≥4.5 | ≥4.5 |
| | | | Fetal cow serum | | ≥4.5 | ≥4.5 |
| Peresal | 2 | | Bovine serum albumin | | ≥4.5 | ≥4.5 |
| | | | | | **Virus Titer** | |
| Water Control - | - | n.d | No soil | | 8.0 | 8.0 |
| | | | Fetal cow serum | | 8.0 | 8.0 |
| | | | Bovine serum albumin | | 8.0 | 8.0 |

| | | | | | | |
|---|---|---|---|---|---|---|
| *1% of INCIDIN PLUS includes 2600 ppm of GLUCOPROTAMIN. 1% of PERESAL includes 450 ppm of peracetic acid. | | | | | | |

The lower concentration of the Peresal (0.5%) with the Incidin Plus was less effective at killing all of the Polio virus. A 1% or 2% solution of Peresal with a 1% solution of Incidin Plus was effective at killing substantially all of the Polio virus.

### Example 3

Example 3 tested the ability of the combination of the amine part and the oxygen part to kill viruses. For this example, various combinations of INCIDIN PLUS and PERESAL were tested against Bovine Virus Diarrhea Virus (BVDV) and Adeno virus (Adenoide 6 Strain) in a suspension test according to the guidelines of the German Society for the Prevention of Viral Disease (DVV).

**Table 3 Log Reductions Against Bovine Virus Diarrhea Virus and Adeno Virus**

| **Product** | **Volume Percent** | **Soiling** | **Virus** | **30 Minutes** | **60 Minutes** |
|---|---|---|---|---|---|
| Incidin Plus | 0.5* | No soil | BVDV | ≥4.0 | ≥4.0 |
| | | Fetal cow serum | | ≥4.0 | ≥4.0 |
| Peresal | 0.5 | Bovine serum albumin | | 2.5 | ≥4.0 |
| Incidin Plus | 1.0 | No soil | | ≥3.0 | ≥3.0 |
| | | Fetal cow serum | | ≥3.0 | ≥3.0 |
| Peresal | 1.0 | Bovine serum albumin | | ≥3.0 | ≥3.0 |
| | | | | **Virus Titer** | |
| Water Control | - | No soil | | 6.5 | 6.5 |
| | | Fetal cow serum | | 6.5 | 6.5 |
| | | Bovine serum albumin | | 6.5 | 6.5 |
| Incidin Plus | 0.5 | No soil | Adeno | ≥4.0 | ≥4.0 |
| | | Fetal cow serum | | ≥4.0 | ≥4.0 |
| Peresal | 0.5 | Bovine serum albumin | | ≥4.0 | ≥4.0 |
| Incidin Plus | 1.0 | No soil | | ≥3.0 | ≥3.0 |
| | | Fetal cow serum | | ≥3.0 | ≥3.0 |
| Peresal | 1.0 | Bovine serum albumin | | ≥3.0 | ≥3.0 |
| | | | | **Virus Titer** | |
| Water Control | - | No soil | | 6.5 | 6.5 |
| | | Fetal cow serum | | 6.5 | 6.5 |
| | | Bovine serum albumin | | 6.5 | 6.5 |

| | | | | | |
|---|---|---|---|---|---|
| *1% of INCIDIN PLUS includes 2600 ppm of GLUCOPROTAMIN. 1% of PERESAL includes 450 ppm of peracetic acid. | | | | | |

The lower concentration (0.5%) of Peresal with the Incidin Plus was less effective at killing all of the BVDV under soiled conditions. A 1% solution of Peresal with a 1% solution of Incidin Plus was effective at killing substantially all of the BVDV and Adeno viruses under all test conditions.

The foregoing summary, detailed description, and examples provide a sound basis for understanding the invention, and some specific example embodiments of the invention. Since the invention can comprise a variety of embodiments, the above information is not intended to be limiting. The invention resides in the claims.

## Claims

1. A two-part antimicrobial system comprising a first part and a second part to yield an aqueous antimicrobial composition, the first part comprising an amine composition selected from the group consisting of Glucoprotamin, N, N-Bis(3-aminopropyl)dodecylamine, and mixtures thereof and the second part comprising an oxygen composition selected from the group consisting of hydrogen peroxide, peracetic acid, peroctanoic acid, and mixtures thereof, wherein the resulting antimicrobial composition maintains efficacy for at least about 15 minutes after mixture.

2. The system of claim 1, wherein the antimicrobial composition maintains efficacy for at least about 60 minutes.

3. The system of claim 1, wherein the amine composition is Glucoprotamin.

4. The system of claim 1, wherein the amine and oxygen compounds are present in the resulting antimicrobial composition in a ratio from about 1:100 to about 100:1.

5. The system of claim 1, wherein the first part, the second part, or both the first and second parts further comprise additional functional ingredients.

6. The system of claim 1, wherein the additional functional ingredients are selected from the group consisting of wetting agents, builders, textual modifiers, film-forming polymers, surfactants, complexing agents, colorants, and mixtures thereof.

7. The system of claim 1, wherein the amine composition is present in the first part in an amount that, when combined with the second part, it is present within the antimicrobial use composition at a concentration ranging from about 0.01 wt.% to about 5 wt.%.

8. The system of claim 1, wherein the oxygen composition is present in the second part in an amount that, when combined with the first part, it is present within the antimicrobial use composition at a concentration ranging from about 0.005 wt.% to about 0.5 wt.%.

9. The system of claim 1, wherein the antimicrobial use composition has a pH in the range of about 2 to about 10.

10. The system of claim 1, wherein both the first part and the second part are independently in the form of an aqueous solution, solid, emulsion, cream, gel, powder, or tablet.

11. An aqueous antimicrobial use composition comprising:
a) an antimicrobial amine composition; and
b) an antimicrobial oxygen composition;
wherein the use composition maintains efficacy for at least about 15 minutes.

12. The composition of claim 11, wherein the use composition maintains efficacy for at least about 60 minutes.

13. The composition of claim 11, wherein the amine composition is Glucoprotamin.

14. The composition of claim 11, wherein the oxygen composition is selected from the group consisting of hydrogen peroxide, peracetic acid, peroctanoic acid, and mixtures thereof.

15. The composition of claim 11, further comprising additional functional ingredients.

16. The composition of claim 11, wherein the additional functional ingredients are selected from the group consisting of wetting agents, builders, textual modifiers, film-forming polymers, surfactants, complexing agents, colorants, and mixtures thereof.

17. The composition of claim 11, wherein the amine composition is present in an amount from about 0.01 wt.% to about 5 wt.%.

18. The composition of claim 11, wherein the oxygen composition is present in an amount from about 0.005 wt.% to about 0.5 wt.%.

19. The composition of claim 11, wherein the composition has a pH in the range of about 3 to about 9.

20. A method of disinfecting a surface comprising:
applying an aqueous antimicrobial use composition to a surface, the antimicrobial use composition comprising:
a) an antimicrobial amine or amine derivative composition selected from the group consisting of Glucoprotamin, N, N-Bis(3-aminopropyl)dodecylamine, and mixtures thereof; and
b) an antimicrobial oxygen composition selected from the group consisting of hydrogen peroxide, peracetic acid, peroctanoic acid, and mixtures thereof,
the use composition present in an amount effective to provide at least a 4 log reduction of *Bacillus subtilis* and maintain efficacy for at least about 15 minutes.

21. A method of disinfecting a surface comprising:
applying an aqueous antimicrobial use composition to a surface, the antimicrobial use composition comprising:
a) an antimicrobial amine or amine derivative composition selected from the group consisting of Glucoprotamin, N,N-Bis(3-aminopropyl)dodecylamine, and mixtures thereof; and
b) an antimicrobial oxygen composition selected from the group consisting of hydrogen peroxide, peracetic acid, peroctanoic acid, and mixtures thereof,
the use composition present in an amount effective to provide at least a 4 log reduction of Polio virus and maintain efficacy for at least about 15 minutes.
